# EUROPÄISCHE PATENTANMELDUNG

(11) **EP 4 725 457 A1**
(43) Veröffentlichungstag der Anmeldung: **15.04.2026**
(21) Anmeldenummer: 25207541.1
(22) Anmeldetag: 08.10.2025
(51) Int. Cl.: A61F 2/38

(54) **VERANKERUNGSEINHEIT ZUR VERANKERUNG EINER KNIEPROTHESENKOMPONENTE AN EINEM BEINKNOCHEN EINES PATIENTEN, PROTHESENKIT**

(30) Priorität: 14.10.2024 DE 102024129689
(71) Anmelder: Aesculap AG, 78532 Tuttlingen (DE)
(72) Erfinder: Hettich, Georg, 79117 Freiburg (DE)
(74) Vertreter: DREISS Patentanwälte PartG mbB

(57) **Zusammenfassung**

Die Erfindung betrifft eine Verankerungseinheit (10) zur Verankerung einer Knieprothesenkomponente (100) an einem Beinknochen, die Verankerungseinheit umfassend: eine Verankerungshülse (12), die eine Hülsenwand (14) aufweist, wobei die Hülsenwand eine Durchgangsöffnung (16) definiert, die zur Aufnahme eines Prothesenschafts (102) ausgebildet ist, wobei die Hülsenwand wenigstens zwei Wandabschnitte (18) aufweist, die separat voneinander ausgebildet und aus einem nicht physiologisch abbaubaren Material gefertigt sind, und wobei die Wandabschnitte durch wenigstens einen Verbindungsabschnitt (20) miteinander verbunden sind, der aus einem physiologisch abbaubaren Material gefertigt ist. Außerdem betrifft die Erfindung ein Prothesenkit.

## Beschreibung

Die vorliegende Erfindung betrifft das Gebiet der Implantattechnik. Insbesondere betrifft die vorliegende Erfindung eine Verankerungseinheit zur Verankerung einer Knieprothesenkomponente an einem Beinknochen eines Patienten, sowie ein Prothesenkit.

Eine Knieprothese (auch als Kniegelenkprothese bezeichnet) ist eine Prothese, die das Kniegelenk vollständig oder teilweise ersetzt. Typischerweise weist eine Knieprothese zwei Knieprothesenkomponenten auf, nämlich eine Femurprothesenkomponente und eine Tibiaprothesenkomponente, wobei die Femurprothesenkomponente in das Femur, also in den Oberschenkelknochen, implantiert wird, und wobei die Tibiaprothesenkomponente in die Tibia, also in das Schienbein, implantiert wird. Derartige Knieprothesenkomponenten weisen typischerweise einen Prothesenschaft auf, der den eigentlichen Gelenkersatzabschnitt der Knieprothesenkomponente trägt.

Bei vorliegenden Knochendefekten wird zusätzlich zu der eigentlichen Knieprothesenkomponente oftmals eine hülsenförmige Verankerungseinheit eingesetzt, die der Verankerung der Knieprothesenkomponente an dem betreffenden Beinknochen dient. Eine solche hülsenförmige Verankerungseinheit wird auch als "Cone" bezeichnet. Ein Cone wird in der Regel im metaphysären Bereich des Beinknochens platziert. Aus diesem Grund bildet die Außenkontur eines Cones oftmals den metaphysären Bereich des Tibia- bzw. des Femurknochens ab und erreicht dadurch eine gute Passform. Ein Cone weist typischerweise eine Durchgangsöffnung auf, die zur Aufnahme eines Prothesenschafts der Knieprothesenkomponente vorgesehen ist. Die derart angeordnete Knieprothesenkomponente kann dann, beispielsweise durch Knochenzement, an dem Cone befestigt werden. Der Cone erleichtert das Anwachsen des Beinknochens, wodurch die Knieprothesenkomponente an dem Beinknochen verankert wird.

Ein solcher Cone ist beispielsweise in der Offenlegungsschrift WO 2024/047127 A1 beschrieben.

In bestimmten Situationen ist es erwünscht, eine angewachsene Knieprothesenkomponente mitsamt des Cones wieder zu entfernen, was auch als Revision bezeichnet wird. Hierzu werden typischerweise zunächst der Knochenzement und dann die Knieprothesenkomponente und der Cone entfernt. Eine Revision wird beispielsweise dann durchgeführt, wenn eine bakterielle Infektion im Bereich des Cones bzw. der Knieprothesenkomponente vorliegt. Der Vorteil der guten Osteointegration wird bei einer Revision zum Nachteil, denn die Entfernung des Cones wird durch das Anwachsen des Beinknochens an den Cone erschwert.

Die Erfindung beschäftigt sich mit der Aufgabe, eine Verankerungseinheit für eine Knieprothesenkomponente bereitzustellen, die im Rahmen einer Revision knochenschonend und mit geringem Zeitaufwand entfernt werden kann.

Ein erster Aspekt der Beschreibung betrifft eine Verankerungseinheit zur Verankerung einer Knieprothesenkomponente an einem Beinknochen eines Patienten. Die Verankerungseinheit weist eine Verankerungshülse auf. Insbesondere wird die Verankerungseinheit durch die Verankerungshülse gebildet, sodass die Verankerungseinheit insgesamt hülsenförmig ausgebildet ist. Die Verankerungseinheit kann aber auch einen oder mehrere weitere Abschnitte zusätzlich zu der Verankerungshülse aufweisen.

Die Verankerungshülse weist eine Hülsenwand auf, die eine Durchgangsöffnung definiert. Insbesondere ist die Hülsenwand in Umfangsrichtung geschlossen, sodass die Hülsenwand die Durchgangsöffnung vollumfänglich umschließt. Die Hülsenwand kann aber auch in Umfangsrichtung unterbrochen sein. Die Durchgangsöffnung ist zur Aufnahme eines Prothesenschafts einer Knieprothesenkomponente ausgebildet.

Die Hülsenwand weist wenigstens zwei Wandabschnitte auf, die separat voneinander ausgebildet und aus einem nicht physiologisch abbaubaren Material gefertigt sind. Die Wandabschnitte sind durch wenigstens einen Verbindungsabschnitt miteinander verbunden, der aus einem physiologisch abbaubaren Material gefertigt ist. Der Verbindungsabschnitt hält die separat voneinander ausgebildeten Wandabschnitte zusammen. Insbesondere sind die beiden Wandabschnitte nur durch einen oder mehrere Verbindungsabschnitte aus einem physiologisch abbaubaren Material miteinander verbunden.

Der Erfinder hat erkannt, dass die zugrundeliegende Aufgabe durch eine wie vorstehend ausgebildete Verankerungseinheit gelöst wird. Nach Implantation der Verankerungseinheit sowie einer zugehörigen Knieprothesenkomponente wird das physiologisch abbaubare Material des Verbindungsabschnitts durch den Körper abgebaut, sodass die durch den Verbindungsabschnitt bereitgestellte Verbindung der Wandabschnitte aufgelöst wird. Die Hülsenwand zerfällt insofern im Anschluss an die Implantation in mehrere separate Teile, die vorzugsweise durch Knochenzement zusammengehalten werden. Nach Entfernung des Knochenzements liegen die Wandabschnitte dann als solche vereinzelt vor, wodurch die Entfernung der Verankerungseinheit, insbesondere der nun vereinzelten Wandabschnitte, erleichtert wird.

Im Rahmen der Offenbarung ist ein "physiologisch abbaubares Material" ein Material, das nach Implantation in einen Beinknochen durch den Körper derart abgebaut wird, dass die strukturelle Integrität eines Abschnitts, der aus dem physiologisch abbaubaren Material gefertigt ist, im Anschluss an die Implantation sukzessive verloren geht, insbesondere über mehrere Wochen oder über mehrere Monate hinweg. Vorzugsweise ist das physiologisch abbaubare Material insgesamt resorbierbar. Alternativ dazu kann lediglich ein Bestandteil des physiologisch abbaubaren Materials resorbierbar sein. Beispielsweise kann das physiologisch abbaubare Material auch eine Matrix aus einem resorbierbaren Bestandteil aufweisen, in der nicht resorbierbare Partikel verteilt vorliegen. Auch bei einem solchen gemischten Material geht die strukturelle Integrität bei Abbau der Matrix sukzessive verloren, sodass es sich um ein physiologisch abbaubares Material handelt.

Im Rahmen der Offenbarung ist ein "nicht physiologisch abbaubares Material" ein Material, das nach Implantation in einen Beinknochen nicht abgebaut wird oder höchstens derart langsam abgebaut wird, dass die strukturelle Integrität eines Abschnitts, der aus einem nicht physiologisch abbaubaren Material gefertigt ist, im Anschluss an die Implantation dauerhaft erhalten bleibt.

Die Hülsenwand weist wenigstens zwei Wandabschnitte auf, die separat voneinander ausgebildet und aus einem nicht physiologisch abbaubaren Material gefertigt sind. Es können auch mehr als zwei solcher Wandabschnitte vorhanden sein. Vorzugsweise sind die Wandabschnitte im Hinblick auf deren Form gleich ausgebildet.

Die Wandabschnitte sind durch wenigstens einen Verbindungsabschnitt miteinander verbunden, der aus einem physiologisch abbaubaren Material gefertigt ist. Es können auch mehr als nur ein solcher Verbindungsabschnitt vorhanden sein.

Vorzugsweise werden die wenigstens zwei Wandabschnitte durch einen oder mehrere Verbindungsabschnitte zusammengehalten, wobei die Wandabschnitte als separate Teile ausgebildet sind. Insbesondere werden die separaten Teile nur durch einen oder mehrere Verbindungsabschnitte zusammengehalten.

Vorzugsweise ist die Verankerungshülse konisch zulaufend ausgebildet. Der Querschnitt der Verankerungshülse nimmt also von einem ersten Längsende der Verankerungshülse zu einem zweiten Längsende der Verankerungshülse ab. Eine derart geformte Verankerungshülse ist für die Platzierung im metaphysären Bereich eines Beinknochens besonders geeignet. Dabei kann die konisch zulaufende Verankerungshülse wahlweise symmetrisch oder asymmetrisch ausgebildet sein. In einer weiteren bevorzugten Ausführungsform ist die Verankerungshülse zylindrisch ausgebildet. Die Verankerungshülse kann auch einen konisch zulaufenden ersten Längsabschnitt und einen zylindrischen zweiten Längsabschnitt aufweisen.

Vorzugsweise weist die Hülsenwand an ihrer Mantelaußenseite wenigstens bereichsweise eine strukturierte Oberfläche auf. Dadurch kann das Anwachsen des Beinknochens an die Verankerungseinheit gefördert werden. Die strukturierte Oberfläche kann beispielsweise Zähne, Rillen und/oder eine Gitterstruktur aufweisen. Eine geeignete Gitterstruktur ist beispielsweise unter der Bezeichnung "Structan Surface" bekannt. Vorzugsweise weisen die Wandabschnitte jeweils wenigstens bereichsweise die strukturierte Oberfläche auf.

In einigen bevorzugten Ausführungsformen ist vorgesehen, dass die Wandabschnitte zueinander versetzt angeordnete Wandsegmente der Hülsenwand sind. Insbesondere sind die Wandsegmente in Umfangsrichtung der Hülsenwand oder in Längserstreckung der Verankerungshülse zueinander versetzt angeordnet. Zueinander versetzt angeordnete Wandsegmente haben den Vorteil, dass sie nach physiologischem Abbau des Materials des Verbindungsabschnitts leicht zugänglich sind und deshalb leicht entfernt werden können. Vorzugsweise sind die Wandsegmente, bezogen auf radiale Blickrichtungen, überlappungsfrei angeordnet.

In einigen bevorzugten Ausführungsformen ist vorgesehen, dass die Wandabschnitte voneinander beabstandet sind, wobei der Verbindungsabschnitt in einem zwischen den Wandabschnitten gebildeten Zwischenraum angeordnet ist. Nach physiogischem Abbau des Materials des Verbindungsabschnitts verbleibt also ein Zwischenraum zwischen den beiden Wandabschnitten. Dadurch wird die Entfernung der beiden Wandabschnitte weiter erleichtert, denn deren Zugänglichkeit wird verbessert. Vorzugsweise sind die beiden Wandabschnitte dabei als zueinander versetzt angeordnete Wandsegmente der Hülsenwand ausgebildet. Vorzugsweise sind die Wandabschnitte und der Verbindungsabschnitt derart ausgebildet, dass die Außenseite des Verbindungsabschnitts mit den Außenseiten der Wandabschnitte flächenbündig ist. Eine solche Ausführung wird durch das Vorsehen des Zwischenraums und die Anordnung des Verbindungsabschnitts in dem Zwischenraum erleichtert. Die Außenseiten sind die von der Durchgangsöffnung abgewandten Seiten der Abschnitte. Vorzugsweise füllt der Verbindungsabschnitt den Zwischenraum zwischen den beiden Wandabschnitten aus. Vorzugsweise ist der Zwischenraum spaltförmig. Vorzugsweise ist der Verbindungsabschnitt nur in dem zwischen den Wandabschnitten gebildeten Zwischenraum angeordnet. Der Verbindungsabschnitt ragt also über die durch die Wandabschnitte definierte Kontur nicht radial hinaus.

In einigen bevorzugten Ausführungsformen ist vorgesehen, dass die Wandabschnitte plattenförmig ausgebildet sind. Bei einem plattenförmigen Element sind die Länge und die Breite des Elements deutlich größer als dessen Dicke. Ein plattenförmiges Element ist also im Wesentlichen zweidimensional erstreckt. Durch die plattenförmige Ausbildung der Wandabschnitte wird durch die Wandabschnitte selbst nach Abbau des Verbindungsabschnitts noch eine effektive Verankerung der Knieprothesenkomponente an dem Beinknochen bewirkt. Die plattenförmigen Wandabschnitte sind vorzugsweise gekrümmt. Eine Verankerungshülse mit gekrümmten Wandabschnitten kann leichter in den metaphysären Bereich eines Beinknochens eingepasst werden.

In einigen bevorzugten Ausführungsformen ist vorgesehen, dass der Verbindungsabschnitt stabförmig ausgebildet ist. Bei einem stabförmigen Element ist die Länge des Elements deutlich größer als dessen Breite und dessen Höhe. Ein stabförmiges Element ist also im Wesentlichen eindimensional erstreckt.

In einigen bevorzugten Ausführungsformen ist vorgesehen, dass die Wandabschnitte in Umfangsrichtung der Verankerungshülse hintereinander angeordnet sind. Der Verbindungsabschnitt erstreckt sich dabei vorzugsweise zwischen den Wandabschnitten und in Längserstreckung der Verankerungshülse.

Vorzugsweise sind mehrere Wandabschnitte und mehrere Verbindungsabschnitte vorhanden, wobei die Wandabschnitte und die Verbindungsabschnitte gemeinsam die in Umfangsrichtung geschlossene Hülsenwand bilden, wobei die Wandabschnitte in Umfangsrichtung der Hülsenwand hintereinander angeordnet sind, und wobei zwischen zwei benachbarten Wandabschnitten stets ein Verbindungsabschnitt angeordnet ist, der die beiden Wandabschnitte miteinander verbindet. Vorzugsweise erstrecken sich dabei die Wandabschnitte jeweils entlang eines Umfangswinkelintervalls von wenigstens 30°. Die Verbindungsabschnitte erstrecken sich vorzugsweise jeweils entlang eines Umfangswinkelintervalls von höchstens 10°.

In einigen bevorzugten Ausführungsformen ist vorgesehen, dass wenigstens einer der Wandabschnitte durch eine Formschlussverbindung mit dem Verbindungsabschnitt verbunden ist. Dadurch kann eine mechanisch robuste Verbindung zwischen dem Wandabschnitt und dem Verbindungsabschnitt erreicht werden.

In einigen bevorzugten Ausführungsformen ist vorgesehen, dass wenigstens einer der Wandabschnitte eine Vertiefung aufweist, und dass der Verbindungsabschnitt zur Ausbildung der Formschlussverbindung in die Vertiefung hineinragt. Die Vertiefung kann in einer in Umfangsrichtung weisenden lateralen Seite des Wandabschnitts ausgebildet sein. Vorzugsweise ist die Vertiefung durch den Verbindungsabschnitt ausgefüllt. Vorzugsweise ist die Vertiefung länglich ausgebildet, beispielsweise als Nut, als Rinne oder als Furche.

In einigen bevorzugten Ausführungsformen ist vorgesehen, dass die Vertiefung einen Hinterschnitt aufweist, und dass der Verbindungsabschnitt zur Ausbildung der Formschlussverbindung den Hinterschnitt hintergreift. Eine solche Ausführung der Formschlussverbindung ist mechanisch besonders robust. Vorzugsweise weist der Verbindungsabschnitt einen schwalbenschwanzförmigen Vorsprung auf, der in die Vertiefung hineinragt und den Hinterschnitt hintergreift.

In einigen bevorzugten Ausführungsformen ist vorgesehen, dass das physiologisch abbaubare Material verformbar ausgebildet ist. Folglich kann die Anordnung der Wandabschnitte relativ zueinander zumindest geringfügig verändert werden, nämlich durch Verformen des Verbindungsabschnitts. Das hat den Vorteil, dass die Anordnung der Verankerungshülse in dem Beinknochen erleichtert wird. Je nach Form der Wandabschnitte und Anordnung der Wandabschnitte relativ zueinander können beispielsweise die Länge und/oder der Durchmesser der Verankerungshülse durch Verformen des Verbindungsabschnitts oder der Verbindungsabschnitte geändert werden. Vorzugsweise ist das physiologisch abbaubare Material elastisch verformbar.

Das nicht physiologisch abbaubare Material ist vorzugsweise steif ausgebildet. Dadurch sind die Wandabschnitte mechanisch robust, können aber im Rahmen der Implantation der Verankerungshülse nicht selbst verformt werden.

In einigen bevorzugten Ausführungsformen ist vorgesehen, dass das physiologisch abbaubare Material ein Polymermaterial umfasst. Ein Polymermaterial ist ein Material, das Makromoleküle aufweist. Die Makromoleküle sind aus einer Wiederholeinheit oder aus mehreren verschiedenen Wiederholeinheiten aufgebaut.

Vorzugsweise ist das Polymermaterial ein Proteinmaterial. Der Verbindungsabschnitt ist also aus einem Proteinmaterial gefertigt. Proteinmaterialien sind sowohl im Hinblick auf ihre mechanischen Eigenschaften als auch im Hinblick auf das Abbauverhalten besonders geeignet. Das Proteinmaterial kann dabei ein natürliches, insbesondere pflanzliches oder tierisches, oder ein synthetisches Proteinmaterial sein. Vorzugsweise weist das physiologisch abbaubare Material als Proteinmaterial Gelatine auf. Gelatine ist insbesondere aufgrund seiner guten Biokompatibilität besonders geeignet.

Vorzugsweise ist das Polymermaterial ein Polyestermaterial. Besonders bevorzugt umfasst das Polyestermaterial Poly-L-Lactid (PPLLA), Poly-Lactid-co-Glycolid (PLGA) und/oder Poly-D,L-Lactid (PDLLA).

In einigen bevorzugten Ausführungsformen ist vorgesehen, dass das physiologisch abbaubare Material ein physiologisch abbaubares Keramikmaterial umfasst. Besonders bevorzugt umfasst das physiologisch abbaubare Keramikmaterial Hydroxylapatit, Alpha-Tricalciumphosphat und/oder Beta-Tricalciumphosphat.

In einigen bevorzugten Ausführungsformen ist vorgesehen, dass das physiologisch abbaubare Material ein physiologisch abbaubares Metall oder eine physiologisch abbaubare Metalllegierung umfasst. Bevorzugte physiologisch abbaubare Metalle sind bspw. Eisen, Zink und Magnesium. Bevorzugte physiologisch abbaubare Metalllegierungen sind magnesiumhaltige Metalllegierungen. Besonders bevorzugt umfasst das physiologisch abbaubare Material eine zink- und calciumhaltige Magnesiumlegierung.

Es sind auch Gemische der zuvor erörterten physiologisch abbaubaren Materialien möglich. Beispielsweise kann das physiologisch abbaubare Material auch ein Gemisch aus einem Polymermaterial und einem Keramikmaterial sein.

In einigen bevorzugten Ausführungsformen ist vorgesehen, dass das nicht physiologisch abbaubare Material ein Metall oder eine Metalllegierung ist. Die Wandabschnitte sind also aus einem Metall bzw. aus einer Metalllegierung gefertigt. Vorzugsweise ist das nicht physiologisch abbaubare Material Titan. Titan ist insbesondere aufgrund seiner hohen Steifigkeit und seiner guten Biokompatibilität besonders geeignet. Vorzugsweise sind die Wandabschnitte durch ein additives Fertigungsverfahren gefertigt, besonders bevorzugt durch selektives Laserschmelzen.

In einigen bevorzugten Ausführungsformen ist vorgesehen, dass die Hülsenwand eine Mantelinnenseite aufweist, und dass an der Mantelinnenseite wenigstens ein Befestigungsanker ausgebildet ist, der von der Mantelinnenseite in die Durchgangsöffnung hineinragt. Wie zuvor erwähnt wird der Prothesenschaft typischerweise durch Knochenzement an der Verankerungseinheit befestigt, wobei der Knochenzement hierzu insbesondere zwischen der Hülsenwand und dem Prothesenschaft in die Durchgangsöffnung eingebracht wird. Der Befestigungsanker wird dabei mit dem Knochenzement umgossen und gewährleistet dadurch eine mechanisch besonders robuste Befestigung. Vorzugsweise weist jeder der Wandabschnitte jeweils wenigstens einen Befestigungsanker auf.

Ein zweiter Aspekt der Beschreibung betrifft ein Prothesenkit. Das Prothesenkit umfasst eine wie vorstehend beschrieben ausgebildete Verankerungseinheit und eine Knieprothesenkomponente, die einen Prothesenschaft aufweist, der in der Durchgangsöffnung der Verankerungseinheit anordenbar ist. Der Prothesenschaft trägt den eigentlichen Gelenkersatzabschnitt der Knieprothesenkomponente.

Hinsichtlich der mit dem Prothesenkit erzielbaren Vorteile wird auf die diesbezüglichen Ausführungen zur Verankerungseinheit verwiesen. Zur weiteren Ausgestaltung des Prothesenkits können die im Zusammenhang mit der Verankerungseinheit beschriebenen Merkmale dienen.

In einigen bevorzugten Ausführungsformen ist vorgesehen, dass das Prothesenkit Knochenzement zur Befestigung der Knieprothesenkomponente an der Verankerungseinheit aufweist.

Im Folgenden wird die Erfindung anhand der Zeichnungen näher erläutert. Dazu zeigen:
- Figur 1: eine perspektivische Darstellung einer Verankerungseinheit zur Verankerung einer Knieprothesenkomponente an einem Beinknochen;
- Figur 2: eine Schnittdarstellung der Verankerungseinheit mit einer darin angeordneten Knieprothesenkomponente; und
- Figur 3: eine Detailansicht einer Hülsenwand der Verankerungseinheit.

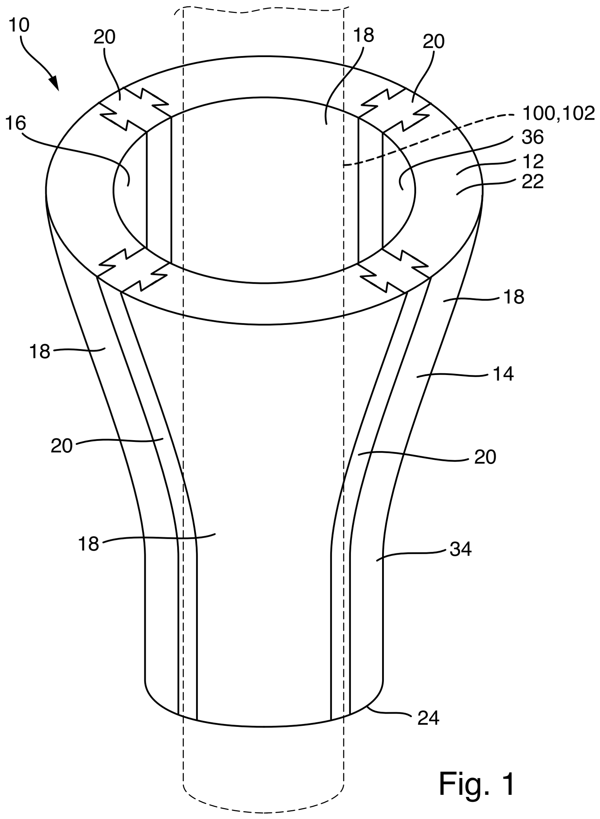

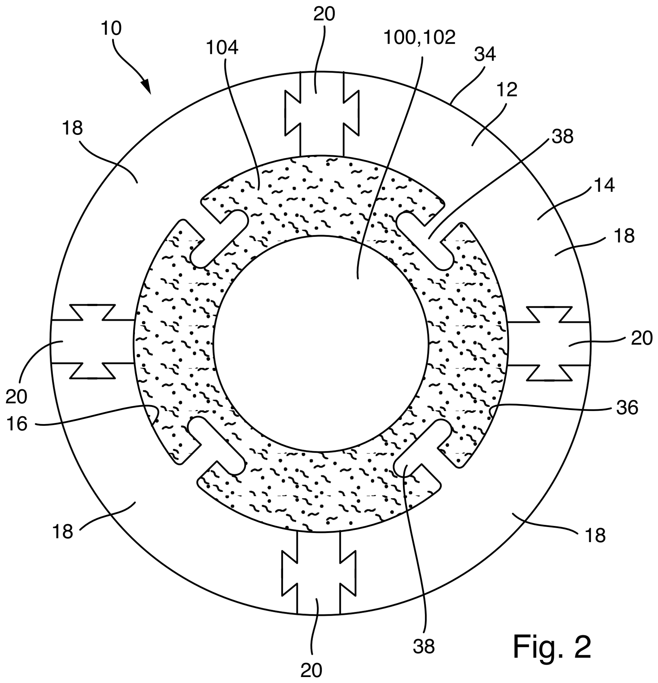

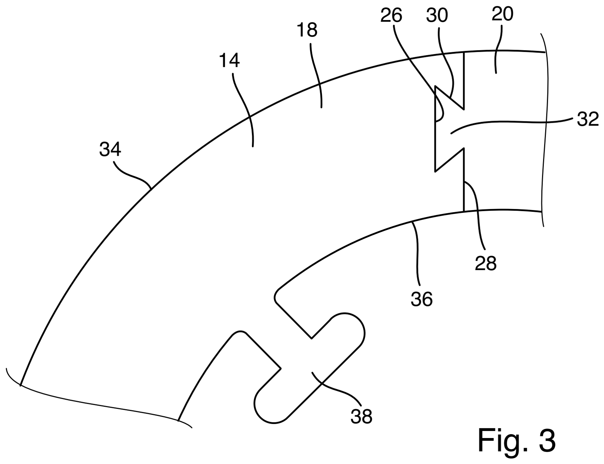

Figur 1 zeigt eine perspektivische Darstellung einer Verankerungseinheit 10. Figur 2 zeigt eine Schnittdarstellung der Verankerungseinheit 10, wobei die Schnittebene senkrecht zu der Längsmittelachse der Verankerungseinheit 10 verläuft. Figur 3 ist eine Detailansicht eines Ausschnitts aus Figur 2.

Die Verankerungseinheit 10 ist zur Verankerung einer Knieprothesenkomponente 100 an einem Beinknochen eines Patienten vorgesehen. Je nach Art der Knieprothesenkomponente 100 kann der Beinknochen das Femur oder die Tibia sein.

Die Verankerungseinheit 10 weist eine Verankerungshülse 12 auf. Vorliegend wird die Verankerungseinheit 10 durch die Verankerungshülse 12 gebildet. Alternativ kann die Verankerungseinheit 10 zusätzlich zu der Verankerungshülse 12 einen oder mehrere weitere, nicht hülsenförmige Abschnitte aufweisen.

Die Verankerungshülse 12 weist eine Hülsenwand 14 auf, die eine Durchgangsöffnung 16 definiert. Vorliegend ist die Hülsenwand 14 umlaufend ausgebildet, also in Umfangsrichtung der Verankerungshülse 12 geschlossen. Die Durchgangsöffnung 16 ist zur Aufnahme eines Prothesenschafts 102 der Knieprothesenkomponente 100 ausgebildet. Ein derart angeordneter Prothesenschaft 102 ist in Figur 1 gestrichelt angedeutet. Wie in Figur 1 zu sehen ist, ragt der Prothesenschaft 102 durch die Verankerungshülse 12 axial hindurch. Der das Kniegelenk nachbildende Abschnitt der Knieprothesenkomponente 100 wird durch den Prothesenschaft 102 getragen, ist der Einfachheit halber aber nicht dargestellt.

Im Rahmen der Implantation der Verankerungseinheit 10 und der Knieprothesenkomponente 100 in einen Beinknochen kann der Prothesenschaft 102 durch Knochenzement 104 an der Verankerungshülse 12 befestigt werden. Durch den Knochenzement 104 kann dabei ein Zwischenraum zwischen dem Prothesenschaft 102 und der Hülsenwand 14 ausgefüllt werden, wie in Figur 2 gezeigt.

Die Verankerungshülse 12 ist vorliegend konisch zulaufend ausgebildet. Eine derart geformte Verankerungshülse 12 ist für die Platzierung im metaphysären Bereich eines Beinknochens besonders geeignet. Vorliegend weist die Verankerungshülse 12 einen kreisförmigen Querschnitt auf, wie in Figur 2 gezeigt.

Die Hülsenwand 14 weist mehrere Wandabschnitte 18 auf, die separat voneinander ausgebildet sind. Die Wandabschnitte 18 sind aus einem nicht physiologisch abbaubaren Material gefertigt. Bei dem in den Figuren dargestellten Ausführungsbeispiel sind genau vier Wandabschnitte 18 vorhanden. Es kann aber auch eine davon abweichende Anzahl an Wandabschnitten 18 vorhanden sein. Bei dem vorliegenden Ausführungsbeispiel sind die Wandabschnitte 18 im Hinblick auf deren Form gleich ausgebildet.

Die Hülsenwand 14 weist außerdem mehrere Verbindungsabschnitte 20 auf. Die separat ausgebildeten Wandabschnitte 18 sind durch die Verbindungsabschnitte 20 miteinander verbunden. Die Verbindungsabschnitte 20 halten also die Wandabschnitte 18 zusammen. Die Verbindungsabschnitte 20 sind aus einem physiologisch abbaubaren Material gefertigt. Bei dem in den Figuren dargestellten Ausführungsbeispiel sind genau vier Verbindungsabschnitte 20 vorhanden. Es kann aber auch eine davon abweichende Anzahl an Verbindungsabschnitten 20 vorhanden sein. Bei dem vorliegenden Ausführungsbeispiel sind die Verbindungsabschnitte 20 im Hinblick auf deren Form gleich ausgebildet.

Im Anschluss an die Implantation der Verankerungseinheit 10 wird das Material der Verbindungsabschnitte 20 sukzessive physiologisch abgebaut. Ein solcher Abbau des Materials kann beispielsweise über mehrere Wochen oder über mehrere Monate hinweg erfolgen. Dadurch geht die strukturelle Integrität der Verbindungsabschnitte 20 verloren und die Wandabschnitte 18 liegen schließlich vereinzelt vor. Gegebenenfalls werden die vereinzelten Wandabschnitte 18 durch den Knochenzement 104 zusammengehalten. Die Vereinzelung der Wandabschnitte 18 im Anschluss an die Implantation hat den Vorteil, dass die Entfernung der Verankerungseinheit 10 erleichtert wird, beispielsweise im Rahmen einer anschließenden infektionsbedingten Revision.

Bei dem in den Figuren dargestellten Ausführungsbeispiel sind die Wandabschnitte 18 zueinander versetzt angeordnete Wandsegmente der Hülsenwand 14. Die Wandabschnitte 18 sind in Umfangsrichtung der Verankerungshülse 12 hintereinander angeordnet und erstrecken sich jeweils entlang der gesamten Länge der Verankerungshülse 12, also von einem ersten Längsende 22 der Verankerungshülse 12 bis zu einem zweiten Längsende 24 der Verankerungshülse 12.

Die Wandabschnitte 18 sind vorliegend plattenförmig ausgebildet und erstrecken sich jeweils entlang eines Umfangswinkelintervalls von etwa 80°. Je nach Anzahl der Wandabschnitte 18 kann auch eine andere Dimensionierung der Wandabschnitte 18 vorgesehen sein.

Wie in den Figuren zu sehen ist, sind die Wandabschnitte 18 voneinander beabstandet, sodass zwischen zwei benachbarten Wandabschnitten 18 stets ein, vorliegend spaltförmiger, Zwischenraum gebildet ist. Die Verbindungsabschnitte 20 sind stabförmig ausgebildet. In jedem der Zwischenräume ist jeweils einer der Verbindungsabschnitte 20 angeordnet. Bei dem in den Figuren dargestellten Ausführungsbeispiel erstrecken sich auch die Verbindungsabschnitte 20 entlang der gesamten Länge der Verankerungshülse 12.

Die Wandabschnitte 18 sind mit den Verbindungsabschnitten 20 formschlüssig verbunden. Die Art der Formschlussverbindung wird im Folgenden mit Bezug auf Figur 3 erläutert. Dort ist eine vergrößerte Detailansicht eines Wandabschnitts 18 und eines Verbindungsabschnitts 20 gezeigt.

Wie in Figur 3 erkenntlich ist, weist der Wandabschnitt 18 eine Vertiefung 26 auf. Die Vertiefung 26 ist in einer in Umfangsrichtung der Hülsenwand 14 weisenden lateralen Seite 28 des Wandabschnitts 18 ausgebildet. Vorliegend ist die Vertiefung 26 länglich ausgebildet und erstreckt sich entlang der gesamten Länge der lateralen Seite 28. Die Vertiefung 26 weist einen Hinterschnitt 30 auf.

Der Verbindungsabschnitt 20 ragt in die Vertiefung 26 hinein und bildet dadurch die Formschlussverbindung aus. Hierzu weist der Verbindungsabschnitt 20 einen schwalbenschwanzförmigen Vorsprung 32 auf, der den Hinterschnitt 30 hintergreift. Der Vorsprung 32 ist vorliegend länglich ausgebildet und erstreckt sich entlang der gesamten Länge des Verbindungsabschnitts 20.

Wie zuvor erwähnt sind die Wandabschnitte 18 aus einem nicht physiologisch abbaubaren Material gefertigt. Vorzugsweise ist das nicht physiologisch abbaubare Material ein Metall oder eine Metalllegierung. Besonders bevorzugt sind die Wandabschnitte 18 aus Titan gefertigt, insbesondere durch ein additives Fertigungsverfahren.

Wie zuvor erwähnt sind die Verbindungsabschnitte 20 aus einem physiologisch abbaubaren Material gefertigt. In diesem Ausführungsbeispiel ist das physiologisch abbaubare Material ein Polymermaterial. Besonders bevorzugt umfasst das Polymermaterial Gelatine oder besteht aus Gelatine. Gelatine ist besonders vorteilhaft aufgrund seiner Biokompatibilität, seiner mechanischen Eigenschaften und seines Abbauverhaltens unter physiologischen Bedingungen.

Es sind aber auch andere physiologisch abbaubare Polymermaterialien möglich, bspw. Polyestermaterialien.

Weitere bevorzugte physiologisch abbaubare Materialien sind physiologisch abbaubare Keramikmaterialien und physiologisch abbaubare Metalle bzw. Metalllegierungen.

In den Figuren ist die Mantelaußenseite 34 der Hülsenwand 14, also die von der Durchgangsöffnung 16 abgewandte Seite der Hülsenwand 14, glatt dargestellt. Abweichend davon ist es bevorzugt, dass die Mantelaußenseite 34 wenigstens bereichsweise eine strukturierte Oberfläche aufweist. Dadurch kann das Anwachsen des Beinknochens an die Verankerungseinheit 10 gefördert werden. Die strukturierte Oberfläche kann beispielsweise Zähne, Rillen und/oder eine Gitterstruktur aufweisen. Eine geeignete Gitterstruktur ist beispielsweise unter der Bezeichnung "Structan Surface" bekannt. Besonders bevorzugt weisen die Wandabschnitte 18 jeweils wenigstens bereichsweise die strukturierte Oberfläche auf.

Wie in den Figuren 2 und 3 zu sehen ist, sind an der Mantelinnenseite 36 der Hülsenwand 14, also an der der Durchgangsöffnung 16 zugewandten Seite der Hülsenwand 14, mehrere Befestigungsanker 38 vorgesehen, die von der Mantelinnenseite 34 in die Durchgangsöffnung 16 hineinragen. Die Befestigungsanker 38 werden beim Einfüllen des Knochenzements 104 vom Knochenzement 104 umschlossen und verstärken dadurch die Verbindung zwischen dem Knochenzement 104 und der Verankerungseinheit 10.

Bei dem vorliegenden Ausführungsbeispiel weist jeder der Wandabschnitte 18 jeweils einen Befestigungsanker 38 auf. Die Befestigungsanker 38 können sich entlang der gesamten Länge der Wandabschnitte 18 erstrecken. Vorliegend erstrecken sich die Befestigungsanker 38 nur entlang eines begrenzten Längsabschnitts der Wandabschnitte 18 und sind deshalb in Figur 1 nicht zu sehen.

## Patentansprüche

1. Verankerungseinheit (10) zur Verankerung einer Knieprothesenkomponente (100) an einem Beinknochen, insbesondere Femur oder Tibia, eines Patienten, die Verankerungseinheit (10) umfassend:
eine Verankerungshülse (12), die eine Hülsenwand (14) aufweist, wobei die Hülsenwand (14) eine Durchgangsöffnung (16) definiert, die zur Aufnahme eines Prothesenschafts (102) einer Knieprothesenkomponente (100) ausgebildet ist, wobei
die Hülsenwand (14) wenigstens zwei Wandabschnitte (18) aufweist, die separat voneinander ausgebildet und aus einem nicht physiologisch abbaubaren Material gefertigt sind, und wobei
die Wandabschnitte (18) durch wenigstens einen Verbindungsabschnitt (20) miteinander verbunden sind, der aus einem physiologisch abbaubaren Material gefertigt ist.

2. Verankerungseinheit (10) nach Anspruch 1, **dadurch gekennzeichnet, dass** die Wandabschnitte (18) zueinander versetzt angeordnete Wandsegmente der Hülsenwand (14) sind.

3. Verankerungseinheit (10) nach einem der vorhergehenden Ansprüche, **dadurch gekennzeichnet, dass** die Wandabschnitte (18) voneinander beabstandet sind, wobei der Verbindungsabschnitt (20) in einem zwischen den Wandabschnitten (18) gebildeten, insbesondere spaltförmigen, Zwischenraum angeordnet ist.

4. Verankerungseinheit (10) nach einem der vorhergehenden Ansprüche, **dadurch gekennzeichnet, dass** die Wandabschnitte (18) plattenförmig ausgebildet sind, und/oder dass der Verbindungsabschnitt (20) stabförmig ausgebildet ist.

5. Verankerungseinheit (10) nach einem der vorhergehenden Ansprüche, **dadurch gekennzeichnet, dass** die Wandabschnitte (18) in Umfangsrichtung der Verankerungshülse (12) hintereinander angeordnet sind.

6. Verankerungseinheit (10) nach einem der vorhergehenden Ansprüche, **dadurch gekennzeichnet, dass** wenigstens einer der Wandabschnitte (18) durch eine Formschlussverbindung mit dem Verbindungsabschnitt (20) verbunden ist.

7. Verankerungseinheit (10) nach dem vorhergehenden Anspruch, **dadurch gekennzeichnet, dass** wenigstens einer der Wandabschnitte (18) eine, insbesondere längliche, Vertiefung (26) aufweist, und dass der Verbindungsabschnitt (20) zur Ausbildung der Formschlussverbindung in die Vertiefung (26) hineinragt.

8. Verankerungseinheit (10) nach dem vorhergehenden Anspruch, **dadurch gekennzeichnet, dass** die Vertiefung (26) einen Hinterschnitt (30) aufweist, und dass der Verbindungsabschnitt (20) zur Ausbildung der Formschlussverbindung den Hinterschnitt (30) hintergreift.

9. Verankerungseinheit (10) nach einem der vorhergehenden Ansprüche, **dadurch gekennzeichnet, dass** das physiologisch abbaubare Material verformbar ausgebildet ist, insbesondere elastisch verformbar.

10. Verankerungseinheit (10) nach einem der vorhergehenden Ansprüche, **dadurch gekennzeichnet, dass** das physiologisch abbaubare Material ein physiologisch abbaubares Polymermaterial, vorzugsweise ein physiologisch abbaubares Proteinmaterial oder ein physiologisch abbaubares Polyestermaterial, ein physiologisch abbaubares Keramikmaterial, ein physiologisch abbaubares Metall und/oder eine physiologisch abbaubare Metalllegierung umfasst.

11. Verankerungseinheit (10) nach einem der vorhergehenden Ansprüche, **dadurch gekennzeichnet, dass** das nicht physiologisch abbaubare Material ein Metall oder eine Metalllegierung ist, insbesondere wobei das nicht physiologisch abbaubare Material Titan aufweist.

12. Verankerungseinheit (10) nach einem der vorhergehenden Ansprüche, **dadurch gekennzeichnet, dass** die Hülsenwand (14) eine Mantelinnenseite (36) aufweist, und dass an der Mantelinnenseite (36) wenigstens ein Befestigungsanker (38) ausgebildet ist, der von der Mantelinnenseite (36) in die Durchgangsöffnung (38) hineinragt.

13. Prothesenkit, umfassend:
a. eine Verankerungseinheit (10) gemäß einem der vorhergehenden Ansprüche; und
b. eine Knieprothesenkomponente (100), insbesondere Femurprothesenkomponente oder Tibiaprothesenkomponente, die einen Prothesenschaft (102) aufweist, der in der Durchgangsöffnung (16) der Verankerungseinheit (10) anordenbar ist.

14. Prothesenkit nach dem vorhergehenden Anspruch, **dadurch gekennzeichnet, dass** das Prothesenkit Knochenzement (104) zur Befestigung der Knieprothesenkomponente (100) an der Verankerungseinheit (10) aufweist.
